# FASCICULE DE BREVET EUROPEEN

(11) **EP 0 651 751 B1**
(45) Date de publication et mention de la délivrance du brevet: **19.02.1997**
(21) Numéro de dépôt: 93916014.9
(22) Date de dépôt: 20.07.1993
(51) Int. Cl.: C07D 487/22, A61K 7/40, A61K 31/40, A61K 31/28, C12P 17/10

(54) **FAMILLE DE SUBSTANCES A NOYAU PORPHINIQUE, LEUR PROCEDE D'OBTENTION A PARTIR DE CYANOPHYCEES, ET LEURS APPLICATIONS EN TANT QUE PRODUITS COSMETIQUES ET EN TANT QUE PRODUITS THERAPEUTIQUES**
STOFFFAMILIE MIT PORPHINRING, IHRE VERFAHREN ZUR HERSTELLUNG AUS CYANOPHYCEEN UND IHRE VERWENDUNGEN ALS KOSMETIKA ODER ARZNEIMITTEL
FAMILY OF PORPHIN RING SUBSTANCES, METHOD OF OBTAINING THEM FROM CYANOPHYCEAE, AND APPLICATIONS AS COSMETICS AND AS THERAPEUTIC PRODUCTS

(30) Priorité: 21.07.1992 FR 9209001
(43) Date de publication de la demande: 10.05.1995
(73) Titulaire: GENERALE DE PREVENTION ET DE LOISIRS, 03200 Vichy (FR)
(72) Inventeur: PEPIN, Denise, F-63400 Chamalières (FR); JEAN, Daniel, F-63270 Vic-le-Comte (FR)
(74) Mandataire: Ores, Irène
(86) Numéro de dépôt international: FR9300734
(87) Numéro de publication internationale: WO9402484

(56) Documents cités:
- EP-A- 0 142 732
- PATENT ABSTRACTS OF JAPAN vol. 7, no. 161 (C-176)1983
- Dictionnaire de la Chimie et de ses applications, 3e édition 1978, C. & R. DUVAL, Technique et Documentation, Paris, page 246.

## Description

La présente invention est relative à une famille de substances à noyau porphinique, à leur procédé d'obtention à partir de plantes chlorophylliennes et notamment de cyanophycées, ainsi qu'à leurs applications en tant que produit cosmétique, notamment dans la lutte contre les effets du vieillissement naturel de la peau et en tant que produit thérapeutique dans certaines affections de la peau (couperose, inflammation, cicatrisation de la peau (brûlures notamment)).

La présente invention a pour objet une famille de substances à noyau porphinique de formules générales Ia et Ib suivantes : 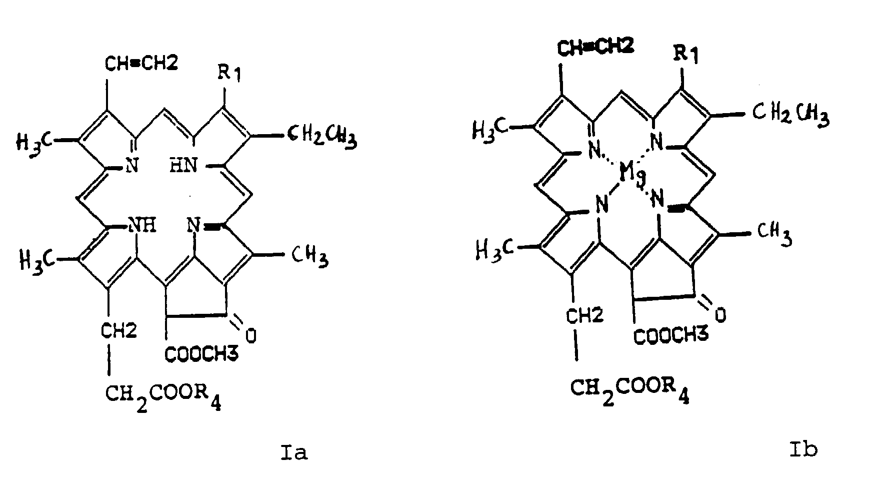 dans lesquelles :
. R₁ est un groupe méthyle ou un groupe CHO, et
. R₄ représente un atome d'hydrogène ou un groupe phytyle,
en tant que produits actifs sur la peau, à la fois en cosmétologie, dans la protection et la lutte contre les effets du vieillissement naturel de la peau et en dermatologie, dans certaines affections de la peau telles que l'inflammation, les blessures, les brûlures ou la couperose, lesquels produits sont sélectionnés parmi :
- les didéhydrochlorophylles de formule Ib, dans lesquelles R₁ représente un groupe CH₃ (didéhydrochlorophylle a) ou un groupe CHO (didéhydrochlorophylle b), et R₄ représente un groupe phytyle,
- les didéhydrochlorophyllides de formule Ib, dans lesquelles R₁ a la même signification que pour les didéhydrochlorophylles et R₄ représente un atome d'hydrogène,
- les didéhydrophaéophytines de formule Ia, dans lesquelles R₁ et R₄ ont la même signification que pour les didéhydrochlorophylles, et
- les didéhydrophaéophorbides de formule Ia, dans lesquelles R₁ et R₄ ont la même signification que pour les didéhydrochlorophyllides.

De tels composés sont généralement utilisés comme pigments colorants ou comme désodorisants.

De manière inattendue, la Demanderesse a trouvé que l'ensemble de ces produits sont actifs sur la restructuration (notamment par leur effet sur la stimulation de la synthèse protéique des cellules) et la protection de la peau (notamment par leur effet anti-protéase), à la fois en cosmétologie (lutte contre les effets du vieillissement de la peau, protection solaire, restructuration des peaux abîmées par les UV et d'autres agents agressifs et lutte contre les vergetures) et en dermatologie, dans certaines affections de la peau (inflammation due aux protéases, couperose, effets déstructurants dus aux corticoïdes, cicatrisation), notamment à des concentrations comprises entre 0,1 et 3 % (en poids).

De manière avantageuse, de telles substances, à la fois lipophiles et hydrophiles pénètrent bien de la couche cornée jusqu'au derme et présentent un effet durable de protection et de restructuration de la peau, alors que les macromolécules habituellement utilisées pour lutter contre les effets du vieillissement naturel de la peau (acide hyaluronique, élastine, collagène ou ADN) ont l'inconvénient majeur d'avoir un effet superficiel et fugace, notamment en raison de l'absence de leur pénétration jusqu'au derme.

De telles substances trouvent application en cosmétologie et en dermatologie, sous la forme de compositions comprenant entre 0,1 et 3 % (en poids) de substances actives, éventuellement associées à d'autres substances actives et/ou excipients convenables.

La présente invention a également pour objet à la fois un procédé d'obtention de l'ensemble de ces substances à noyau porphinique et un procédé d'obtention spécifique de certaines de ces substances, à partir des parties vertes de plantes chlorophylliennes et notamment de cyanophycées fraîches lyophilisées.

Le procédé d'obtention de l'ensemble de ces substances à noyau porphinique de formule Ia et Ib, comprend les étapes suivantes :
(1) au moins une macération à froid des parties de plantes chlorophylliennes et notamment de cyanophycées lyophilisées dans un solvant anhydre, choisi parmi le chloroforme, le chlorure de méthylène, l'éthanol absolu et le méthanol absolu, pour l'obtention d'un extrait contenant à la fois les didéhydrochlorophylles, les didéhydrochlorophyllides, les didéhydrophaéophorbides et les didéhydrophaéophytines, et
(2) séparation chromatographique de ces différentes substances en présence d'un solvant de polarité croissante.

Le solvant à polarité croissante est notamment choisi parmi l'acétone, le méthanol et l'éthanol plus ou moins aqueux.

Selon un mode de mise en oeuvre avantageux de ce procédé, il comprend, préalablement à l'étape (1), un prétraitement de la plante chlorophyllienne, comprenant au moins une macération desdites plantes fraîches ou préalablement lyophilisées, dans un solvant très peu polaire, puis le séchage desdites plantes prétraitées.

Ces plantes prétraitées sont directement utilisées pour l'étape (1) ci-dessus.

On entend, au sens de la présente invention, par séchage, aussi bien un séchage classique (chaleur et aération) qu'un séchage par lyophilisation.

Le solvant peu polaire solvant peu polaire est choisi parmi l'éther de pétrole, le cyclohexane, l'hexane et le tétrachlorure de carbone.

En variante, on peut séparer spécifiquement et sélectivement chacune des sous-familles de substances actives définies ci-dessus.

Pour une séparation spécifique des didéhydrochlorophyllides de formule Ib, le procédé comprend :
(1) au moins une macération de 24 h à une semaine des parties vertes de plantes chlorophylliennes et notamment de cyanophycées lyophilisées dans un solvant aqueux constitué de 20 à 60 % d'eau et d'un solvant miscible à l'eau choisi dans le groupe constitué par l'acétone, l'alcool éthylique et le méthanol,
(2) séchage des plantes ainsi traitées,
(3) au moins une macération à froid des plantes obtenues en (2) dans un solvant anhydre, pour l'obtention d'un extrait contenant essentiellement des didéhydrochlorophyllides et
(4) séparation chromatographique des didéhydrochlorophyllides.

Pour une séparation spécifique des didéhydrophaéophorbides de formule Ia, le procédé comprend soit :
(1) une macération pendant 24 heures à une semaine à température ambiante d'un extrait de didéhydrochlorophyllides selon la revendication 6 dans un solvant organique auquel on a ajouté un acide miscible (acide formique, acide acétique ou autre acide à une concentration correspondant à un pH compris entre 2 et 4, en référence à une solution aqueuse) et
(2) séparation chromatographique des didéhydrophaéophorbides.
soit :
(1) au moins une macération à température ambiante de 24 h à 48 h des parties vertes de plantes chlorophylliennes et notamment de cyanophycées lyophilisées dans un solvant miscible à l'eau tel que l'acétone, auquel on a ajouté un acide miscible (acide formique, acide acétique ou autre acide à une concentration correspondant à un pH compris entre 2 et 4, en référence à une solution aqueuse)
(2) séchage des plantes ainsi traitées,
(3) au moins une macération à froid des plantes obtenues en (2) dans un solvant anhydre, pour l'obtention d'un extrait contenant essentiellement des didéhydrophaéophorbides et
(4) séparation chromatographique des didéhydrophaéophorbides.

Pour une séparation spécifique des didéhydrophaéophytines de formule Ia, le procédé comprend :
(1) une macération pendant une période de 24 h à une semaine, à température ambiante d'un extrait de didéhydrochlorophylles selon la revendication 2, dans un solvant organique auquel on a ajouté un acide miscible (acide formique, acide acétique..., à une concentration correspondant à un pH compris entre 2 et 4, en référence à une solution aqueuse) et
(2) séparation chromatographique des didéhydrophaéophytines.

Les procédés de séparation sélectifs peuvent comprendre, préalablement à l'étape (1), un prétraitement de la plante chlorophyllienne, comprenant au moins une macération desdites plantes fraîches ou préalablement lyophilisées, dans un solvant très peu polaire, puis le séchage desdites plantes prétraitées.

Selon un mode de réalisation avantageux de l'invention, ladite cyanophycée est une souche de *Plectonema.*

Les différentes substances obtenues peuvent être utilisées seules ou en mélange, selon l'effet recherché.

La présente invention a également pour objet une souche de *Plectonema,* caractérisée en ce qu'elle comprend l'ensemble des substances actives sur la peau telles que définies ci-dessus.

Selon un mode de réalisation avantageux de l'invention, une souche *Plectonema* préférée a été déposée auprès de la Collection Nationale de Culture de Microorganismes de l'INSTITUT PASTEUR, 25 rue du Docteur Roux, 75724 PARIS Cédex 15 (FRANCE), en date du 29 mai 1991 sous le n° I-1101.

Elle se présente sous forme de masses gélatineuses de couleur vert émeraude.

Les substances conformes à l'invention peuvent être utilisées pour l'obtention d'un médicament destiné à l'utilisation dermatologique ou à titre de produit cosmétique.

La présente invention a également pour objet une méthode de traitement esthétique de l'Homme dans la protection de la peau et la lutte contre le vieillissement naturel de la peau, comprenant l'administration d'au moins une substance conforme à l'invention, éventuellement associée à au moins un véhicule acceptable.

Selon un mode de réalisation avantageux de l'invention, lesdites substances de formules Ia et Ib sont sous la forme d'une composition comprenant 0,1 à 3 % en poids desdites substances.

Outre les dispositions qui précèdent, l'invention comprend encore d'autres dispositions, qui ressortiront de la description qui va suivre, qui se réfère à des exemples de mise en oeuvre du procédé objet de la présente invention.

Dans ces exemples les termes didéhydrochlorophylle, didéhydrochlorophyllide, didéhydrophaéophytine et didéhydrophaéophorbide sont respectivement remplacés par chlorophylle, chlorophyllide, phaéophytine et phaéophorbide.

Il doit être bien entendu, toutefois, que ces exemples sont donnés uniquement à titre d'illustration de l'objet de l'invention, dont ils ne constituent en aucune manière une limitation.

### EXEMPLE 1 : Effets de restructuration et de protection de la peau des substances conforme à l'invention.

* Accroissement de la résistance du collagène aux effets de la collagénase :
- Mise en évidence :
On répartit dans quatre boîtes de Pétri de 8 cm de diamètre 20 ml d'une solution à 1 % d'agarose dans du tampon Tris pH 8,8, contenant en suspension 20 mg de collagène fibreux insoluble et 0, 0,1, 0,3 et 0,5 ml d'une solution à 5 % de produit à tester selon les boîtes, soient des concentrations de 0, 0,025 %, 0,075 %, 0,125 %. Comme la plupart des produits à tester ne sont pas solubles dans le tampon, on part d'une solution mère dans du diméthylsulfoxyde (DMSO). Cette répartition se fait à 45°C.
Après solidification de l'agarose, on perfore 5 puits de 3 mm de diamètre dans le gel grâce à un emporte-pièce. Dans ces 5 puits, on place 50 µl d'une solution de collagénase pancréatique dans du tampon Tris pH 8,8 à une concentration de 10 mg/ml (3800 U/ml).
Les boîtes de Pétri sont incubées 15 heures à 37°C, puis le gel est coloré par une solution à 0,25 % d'acide picrique à 0,25 % pendant 3 min, puis une solution de Rouge Sirius à 0,1 % pendant 5 min. L'excès de coloration est éliminé par lavage à l'eau.
- Résultats :

| Produit testé | 0,025 % | 0,05 % | 0,075 % |
|---|---|---|---|
| Chlorophylle a, pureté : 65 % | 5 % | 22 % | 28 % |
| Chlorophyllide a, pureté : 74 % | 3 % | 34 % | 65 % |
| Phaéophytine a, pureté : 54 % | 0 % | 5 % | 9 % |
| Phaéophorbide a, pureté : 82 % | 2 % | 8 % | 12 % |

On évalue l'action enzymatique en mesurant le diamètre des zones d'hydrolyse du collagène fibreux.
Le groupe chlorophylle-chlorophyllide est le plus actif, ce qui suggère que la présence de magnésium dans la structure a une grande importance.
* Effet d'inhibition de la collagénase :
- Mise en évidence :
On répartit dans quatre boîtes de Pétri de 8 cm de diamètre 20 ml d'une solution à 1 % d'agarose dans du tampon Tris pH 8,8, contenant en suspension 20 mg de collagène fibreux insoluble. Cette répartition se fait à 45°C.
Après solidification de l'agarose, on perfore 5 puits de 3 mm de diamètre dans le gel grâce à un emporte-pièce. Dans ces 5 puits, on place 25 µl d'une solution de collagénase pancréatique dans du tampon Tris pH 8,8 à des concentrations de : 10 mg/ml (3800 U/ml) et 25 µl d'une solution de la substance à tester dans le même tampon à une concentration de 4 %, 3 %, 2 %, 1 % et 0 %. Comme la plupart des produits à doser ne sont pas solubles dans le tampon, on part d'une solution mère dans du diméthylsulfoxyde. Les boîtes de Pétri sont incubées 15 heures à 37°C, puis le gel est coloré par une solution à 0,25 % d'acide picrique à 0,25 % pendant 3 min, puis une solution de Rouge Sirius à 0,1 % pendant 5 min. L'excès de coloration est éliminé par lavage à l'eau.
- Résultats :

| Produit testé | 1 % | 2 % | 3 % | 4 % |
|---|---|---|---|---|
| Chlorophylle a, pureté : 65 % | 2 % | 3 % | 3 % | 4 % |
| Chlorophyllide a, pureté : 74 % | 3 % | 6 % | 8 % | 9 % |
| Phaéophytine a, pureté : 54 % | 0 % | 0 % | 0 % | 2 % |
| Phaéophorbide a, pureté : 82 % | 2 % | 2 % | 2 % | 6 % |

Dans ce test, les substances les plus performantes sont les mêmes que dans le test précédent (chlorophylle-chlorophyllide) ; de plus, ces substances présentent un effet de renforcement de la résistance du collagène contre les effets de la collagénase, alors que l'enzyme elle-même est peu atteinte par ces substances.
* Inhibition de l'élastase pancréatique :
- Mise en évidence :
L'activité de l'élastase pancréatique est déterminée sur un substrat synthétique. On constate que les produits décrits ci-dessus ont un effet sur cette enzyme alors que le substrat est un dérivé de peptide de faible poids moléculaire : le N succinyl N ala-ala-ala-p nitroanilide.
Solution substrat : solution du substrat ci-dessus à 1 % dans du tampon phosphate pH 7,6.
Solution enzymatique : élastase pancréatique à (1000 U/ml) dans le tampon pH 7,6.
Solvant DMSO à 50 % dans de l'eau distillée.
Solution à tester : substance à tester en solution dans le solvant à 0,1 %.
Dans un premier tube à essai (blanc), on place :
2,2 ml de tampon pH 7,6
0,3 ml de solution enzymatique
0,5 ml de solvant.

Dans un deuxième tube à essai (essai), on place :
1 ml de solution substrat
1,2 ml de tampon pH 7,6
0,3 ml de solution enzymatique
0,5 ml de solution à tester.

Dans un troisième tube à essai, on place :
1 ml de solution substrat
1,2 ml de tampon pH 7,6
0,3 ml de solution enzymatique
0,5 ml de solvant.

On mesure la D.O. de la solution essai contre la solution blanc à 410 nm. On note la valeur toutes les 30 secondes pendant 3 minutes. On réalise la même mesure pour la solution témoin.
L'activité de l'enzyme est définie par la vitesse d'augmentation de D.O. du témoin.
L'activité de la solution inhibitrice à tester se définit comme une diminution relative de l'activité de l'enzyme.
- Résultats :

| Produit testé | |
|---|---|
| Chlorophylle a, pureté : 65 % | 12 % |
| Chlorophyllide a, pureté : 74 % | 28 % |
| Phaéophytine a, pureté : 54 % | 9 % |
| Phaéophorbide a, pureté : 82 % | 17 % |

Dans ce test, qui est un test relatif à une inhibition enzymatique vraie, puisque le substrat est artificiel et de structure assez éloignée des substrats naturels de l'enzyme, on constate que les molécules les plus actives en tant qu'inhibiteurs, sont celles dépourvues de radical phytile ; la présence du magnésium n'apparaît pas comme essentielle, pour cette fonction particulière, d'où l'intérêt de compositions comprenant plusieurs des substances actives définies ci-dessus.
* Effet des substances décrites sur la synthèse des protéines totales par des fibroblastes en culture (restructuration) :
- Mise en évidence :
Une étude de cytotoxicité est réalisée au préalable pour déterminer les concentrations maximales de test des différentes substances concernées.
Cellules utilisées : fibroblastes humains MRC5, au 28ème passage.
Les cellules sont ensemencées sur une plaque à 96 puits à raison de 2.10⁵ cellules par puits environ et mises en culture dans du milieu minimum de Eagle (MEM) additionné de 1 % de glutamine 200 mM, 1 % de solution de vitamines, 100 U/ml de pénicilline, 100 U/ml de streptomycine et 2 % de sérum de veau foetal.
La solution à tester est ajoutée à raison de 100 µl par puits, à la concentration de 50 µg/ml.
Les cellules sont incubées à 37°C sous 5 % d'atmosphères de CO₂. On prélève le milieu de culture après 48, 72, 96 et 120 heures de culture.
Les protéines totales synthétisées sont déterminées après coloration des surnageants par le bleu de Coomassie et mesure de la D.O. à 595 nm. L'augmentation de la synthèse protéique est mesurée par rapport au témoin (solvant de la substance à tester).
- Résultats :

| Produit testé | |
|---|---|
| Chlorophylle a, pureté : 65 % | 15 % |
| Chlorophyllide a, pureté : 74 % | 23 % |
| Phaéophytine a, pureté : 54 % | 4 % |
| Phaéophorbide a, pureté : 82 % | 1 % |

Le groupe chlorophylle-chlorophyllide est le plus actif, ce qui montre l'importance du magnésium dans la structure, pour cette activité.
* Effet antiradicalaire :
Deux techniques peuvent être utilisées pour mettre en évidence l'effet antiradicalaire *in vitro* des produits concernés.
- Mise en évidence par la méthode enzymatique :
- Substrat :: EDTA : 0,05 g
Xanthine : 0,0125 g
Cytochrome c : 0,02 g
Tampon phosphate pH 7,5 : 200 ml.
- Enzyme :: xanthine oxydase à 1 % dans le tampon.
- Solvant :: DMSO à 50 % dans de l'eau distillée.
- Substance antiradicalaire :: 0,5 % dans le solvant.
- Solution blanc :: substrat 2 ml
tampon pH 7,5 : 0,5 ml
solvant : 0,5 ml
- Solution témoin :: substrat : 2 ml
solution enzymatique : 0,5 ml
solvant : 0,5 ml.
- Solution essai :: substrat : 2 ml
solution enzymatique : 0,5 ml
solution antiradicalaire (substance selon l'invention) : 0,5 ml.

On mesure la D.O. de la solution témoin contre la solution blanc à 550 nm toutes les 30 secondes pendant 3 min.
Puis on mesure la D.O. de la solution essai contre la solution blanc toutes les 30 secondes pendant 30 min.
On détermine l'effet antiradicalaire comme le rapport des deux vitesses mesurées.
- Résultats :

| Produit testé | |
|---|---|
| Chlorophylle a, pureté : 65 % | 75 % |
| Chlorophyllide a, pureté : 74 % | 61 % |
| Phaéophytine a, pureté : 54 % | 38 % |
| Phaéophorbide a, pureté : 82 % | 21 % |

Dans ce test, on constate une relative homogénéité des actions des différentes substances, contrairement aux tests précédents ; ici, c'est donc la structure commune qui doit jouer le rôle principal dans cette action.
- Mise en évidence par la méthode chimique :
Réactif : 1,1-diphényl-2-picrylhydrazyle : 2,5 mg dans 100 ml de méthanol.
Solvant des substances antiradicalaires : méthanol.
Solution antiradicalaire : 2 % dans du méthanol.
Ce produit est un radical libre stable pouvant être dégradé par des substances antiradicalaires.
On réalise un blanc pour le témoin et un blanc pour l'essai :
- Blanc témoin :: tampon phosphate pH 7 : 1,5 ml
méthanol : 3 ml
- Témoin :: solution réactif : 3 ml
tampon phosphate pH 7 : 1,5 ml
- Blanc essai :: tampon phosphate pH 7 : 1,35 ml
méthanol : 3,15 ml
- Essai :: solution réactif : 3 ml
tampon phosphate pH 7 : 1,35 ml
solution d'antiradicalaire : 0,15 ml.

On agite à 25°C pendant 5 min. Puis on extrait par 4 ml de toluène pendant 30 secondes. On filtre sur un papier séparateur de phases et on lit la D.O. de la solution de toluène à 519 nm contre la solution de toluène obtenue pour le blanc correspondant. On calcule le pourcentage de réactif disparu.
- Résultats :

| Produit testé | |
|---|---|
| Chlorophylle a, pureté : 65 % | 84 % |
| Chlorophyllide a, pureté : 74 % | 83 % |
| Phaéophytine a, pureté : 54 % | 20 % |
| Phaéophorbide a, pureté : 82 % | 33 % |

Le groupe chlorophylline-chlorophyllide est le plus actif, ce qui indique que la présence du magnésium dans la structure, a une grande importance, contrairement au test précédent.

### EXEMPLE 2 : Procédé d'obtention des substances actives.

### I Obtention de l'ensemble des substances actives.

A. On fait macérer trois fois successivement 10 g d'algues lyophilisées dans 200 ml d'éther de pétrole à +4°C, pour éliminer différentes parties lipidiques et notamment les carotènes. Les résidus d'évaporation des macérations sont éliminés.

L'algue est alors séchée à l'obscurité et elle est soumise à trois macérations successives dans 200 ml d'acétone à +4°C.

Les solutions acétoniques sont alors réunies et évaporées à sec sous pression réduite à +20°C. Le résidu obtenu est de 830 mg.

100 mg de ce résidu sont alors redissous dans 1 ml d'acétone et déposées en ligne sur une plaque de gel de silice préparative 20 X 20 cm de 0,5 mm d'épaisseur.

On réalise le développement du chromatogramme sur 15 cm dans le solvant :

| | |
|---|---|
| Benzène | 60 |
| Dichlorométhane | 15 |
| Acétone | 2 |
| Méthanol | 1 |

Les quatre composants chlorophylliens principaux se situent aux Rf suivants :

| | |
|---|---|
| Chlorophylle a | 0,68 |
| Chlorophyllide a | 0 |
| Phaéophytine a | 0,52 |
| Phaéophorbide a | 0,12 |

Les tâches sont visibles à la lumière naturelle. Elles sont séparées de la plaque par grattage et reprises par 5 ml d'acétone.

Après filtration, l'acétone est évaporée à sec sous pression réduite. On obtient :

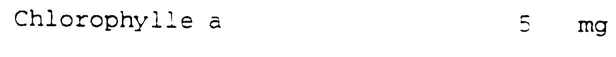

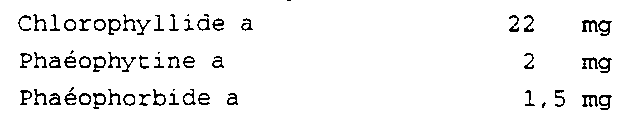

### B. Autre procédé de préparation :

On fait macérer trois fois successivement 1000 g d'algues lyophilisées dans 20 l d'éther de pétrole à +4°C, pour éliminer différentes parties lipidiques et notamment les carotènes. Les résidus d'évaporation des macérations sont éliminés.

L'algue est alors séchée à l'obscurité et elle est soumise à trois macérations successives dans 20 l d'acétone à +4°C.

Les solutions acétoniques sont alors réunies et évaporées à sec sous pression réduite à +20°C. Le résidu obtenu est de 80 g environ, soit un rendement approximatif de 8 %. Le résidu acétonique est alors repris par 500 ml d'acétone et mélangé à 200 g de silice 60 pour chromatographie. L'acétone est alors évaporée sous pression réduite à +20°C jusqu'à ce que la silice soit complètement sèche.

On place alors cette silice, sur laquelle on a fixé l'extrait acétonique, sur une colonne de chromatographie de 50 cm de long et de 20 cm de diamètre remplie de silice 60 dans du chloroforme. Le dépôt est effectué de façon à ce que la jonction entre silice chargée et silice pure soit parfaitement plane. On place ensuite sur le sommet de la silice un disque de papier filtre de 20 cm de diamètre et des billes de verre pour le maintenir. On élue alors par un gradient linéaire de chloroforme acétone à un débit de 10 ml par minute à raison d'une augmentation de la proportion d'acétone de 1 % par minute. On obtient donc 100 % d'acétone en 100 minutes.

Les différentes fractions colorées éluées sont analysées en chromatographie sur couche mince. On peut ainsi recueillir la chlorophylle a, la phaéophytine a, la phaéophorbide a et la chlorophyllide a.

On obtient ainsi 6,2 g de chlorophylle a, 28 g de chlorophyllide a, 1 g de phaéophytine a et 2,2 g de phaéophorbide a.

Il Obtention sélective des différentes substances actives.
- Les chlorophyllides peuvent être produites plus abondamment par macération préalable de 48 heures de l'algue dans du méthanol aqueux à 60 %. Le procédé décrit en A ou en B est ensuite réalisé sur l'algue ainsi traitée et séchée.
- Les phaéophorbides peuvent être produites plus abondamment par traitement pendant une période de 48 heures à température ordinaire de l'algue par une solution acétonique contenant 2 % d'acide formique. Le procédé décrit en A ou en B est ensuite réalisé sur l'algue ainsi traitée et séchée.
- L'obtention des phaéophytines peut être réalisée par traitement pendant une période de 24 heures à une semaine à température ordinaire d'une solution de chlorophylles dans un solvant organique auquel on a ajouté un acide miscible tel que l'acide formique, l'acide acétique ou autre acide à une concentration correspondant à un pH de 2 à 4 environ, si le même acide était placé en solution aqueuse.

Préalablement, ces plantes peuvent être prétraitées par au moins une macération desdites plantes fraîches ou préalablement lyophilisées, dans un solvant très peu polaire, puis le séchage desdites plantes prétraitées.

### EXEMPLE 3 : Exemples de formulation cosméto-dermatologiques contenant les substances conformes à l'invention.

Pour chaque formule, on peut admettre que la concentration globale en produit actif quel qu'il soit est comprise entre 0,1 à 3 % en poids.
* Crème :
   - Mélange de glycérides partiels, d'alcool gras, d'ester cireux et d'alcools gras éthoxylés 10
   - Myristate d'isopropyle 8
   - Huile de vaseline fluide 3
   - Mélange de cyclométhicone et de diméthicone 3
   - Glycérine 10
   - Mélange de polyacrylamide, d'alcool gras éthoxylé et d'isoparaffine 1
   - Mélange de Nipa esters (28 %) dans du phénoxyéthanol 0,5
   - Parfum 0,2
   - Soude à 1N 0,6
   - Chlorophyllide a 1
   - Eau désionisée Q.S.P. 100
* Lait :
   - Glycéryl stéarate 8
   - Monostéarate de glycérine de polyoxyéthylène 3
   - Myristate d'isopropyle 8
   - Stéarate d'isooctyle 2
   - 2-octyl dodécanol 5
   - Mélange de cyclométhicone et de diméthicone 3
   - Glycérine 5
   - Mélange de Nipa esters (28 %) dans du phénoxyéthanol 0,5
   - Parfum 0,2
   - Soude à 1N 0,5
   - Extrait acétonique avant chromatographie tel que décrit dans le protocole précédent 0,5
   - Eau désionisée Q.S.P. 100
* Lotion :
   - 2-hydroxyalcoxylate d'alcool gras 2
   - Polyolester d'acide gras 2
   - Glycérine 3
   - Alcool éthylique à 96 % 20
   - Hydroxyéthylcellulose 0,6
   - Parfum 0,3
   - Chlorophylle a 0,2
   - Eau désionisée Q.S.P. 100
* Gel :
   - Acide polyacrylique réticulé 0,5
   - 2-hydroxyalcoxylate d'alcool gras 2
   - Polyolester d'acide gras 2
   - Glycérine 2
   - Lessive de soude à 35 % 0,43
   - Mélange de Nipa esters (28 %) dans du phénoxyéthanol 0,5
   - Parfum en conformité avec les normes IFRA-RIFM 0,2
   - Extrait brut acétonique tel que décrit dans le protocole précédent 3
   - Eau désionisée Q.S.P. 100

### EXEMPLE 4 : Souche de Plectonema sp. dénommée VY1, conforme à l'invention : aspect, composition et méthode de culture :

La souche axénisée (élimination de tout contaminant bactérien) se présente sous forme de masses gélatineuses de couleur vert émeraude et peut être conservée de différentes façons :
* Conservation dans l'azote liquide :
la souche est simplement immergée sans précaution particulière dans l'azote liquide dans un tube adapté.
* Conservation en culture sur milieu liquide :
- Composition du milieu :

| | |
|---|---|
| NaNO₃ | 1,5 g/l |
| KH₂PO₄.3H₂O | 0,04 g/l |
| MgSO₄.7H₂O | 0,075 g/l |
| CaCl₂.2H₂O | 0,036 g/l |
| Acide citrique | 0,006 g/l |
| Citrate d'ammonium-fer (III) | 0,006 g/l |
| Magnésium-Titriplex.2H₂O | 0,001 g/l |
| Solution de trace de métaux (cf. ci-dessous) | 1 ml/l |
| Hydrolysat de caséine | 0,25 g/l |
| Eau désionisée | q.s. 1000 ml |
| pH après stérilisation | 7,4 |

- Composition de la solution de traces de métaux :

| | |
|---|---|
| H₃BO₃ | 2,86 g/l |
| MnCl₂.4H₂O | 1,81 g/l |
| ZnSO₄.7H₂O | 0,222 g/l |
| Na₂MoO₄.2H₂O | 0,390 g/l |
| CuSO₄.5H₂O | 0,079 g/l |

La culture doit être aérée constamment par une agitation douce et éclairée par des rampes lumineuses fluorescentes de type lumière du jour à raison de 10 µE.m²/s en alternance jour-nuit (12-12).
La température de culture peut être comprise entre 20°C et 45°C.
* Conservation en culture sur milieu solide :
Le milieu de culture solide est préparé en ajoutant 0,6 % d'agarose et 5 mM de bicarbonate de sodium au milieu liquide complet décrit ci-dessus.
Les conditions de culture, température et éclairement sont les mêmes que pour le milieu liquide.
* Récolte et préparation :
La matière première, constituée principalement de *Plectonema sp.* est récoltée au griffon de la source Boussange de Vichy. Cette algue doit être propre de tous débris et d'une coloration vert émeraude franche.
L'algue est aussitôt congelée à -18°C si elle doit être stockée ou transportée.
L'algue est ensuite lyophilisée ou traitée aussitôt après décongélation.
* Composition chimique de l'algue :
- Composants banaux :
. Glucides :
Les glucides contenus dans l'algue sont sous forme libre et sous forme condensée. On trouve principalement du mannose et du glucose.
. Lipides :
Les lipides totaux représentent de 2 à 12 % du poids sec de l'algue. On note la présence d'alcool triterpénique et de vitamine A.
Les acides gras principaux participant à la composition de l'algue sont les suivants :

| | |
|---|---|
| Acide palmitique | environ 50 % des acides gras totaux |
| Acide oléique | environ 15 % des acides gras totaux |
| C18:1ω7 | environ 10 % des acides gras totaux |
| C18:2ω6 | environ 4 % des acides gras totaux |
| Acide stéarique | environ 3 % des acides gras totaux |
| C16:1ω7 | environ 1 % des acides gras totaux. |

. Protides :
Les protides occupent une place très importante dans la composition chimique de l'algue (de 20 à 70 % du poids sec).
. Acides aminés :
Les acides aminés libres représentent de 1 à 7 % du poids sec de l'algue.
On trouve essentiellement : alanine, phénylalanine, acide glutamique, valine. . Pigments :
Pigments de structures porphiniques : chlorophylles, chlorophyllides, phaéophytines, phaéophorbides.
Pigments de type caroténoïdiques : β-carotène, zéaxanthine, autres caroténoïdes non identifiés.
Phycocyanine : pigment bleu instable de nature protéique et caractéristique des cyanophycées.

## Revendications

1. Famille de substances à noyau porphinique de formules générales Ia et Ib suivantes : 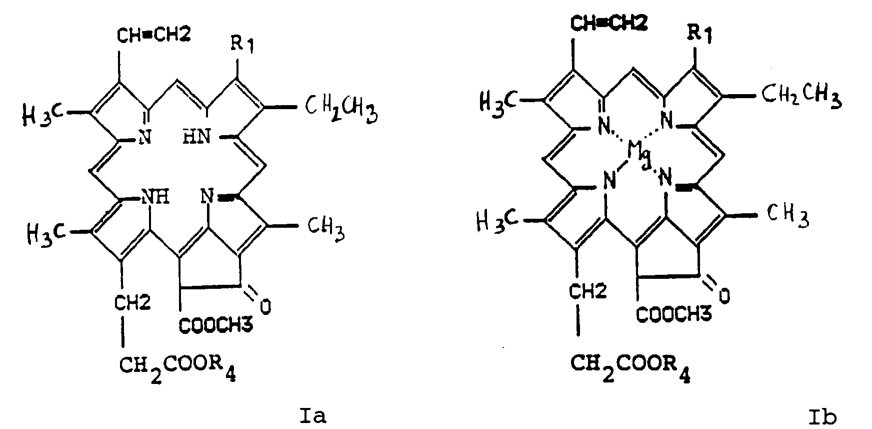 dans lesquelles :
. R₁ est un groupe méthyle ou un groupe CHO, et
. R₄ représente un atome d'hydrogène ou un groupe phytyle,
en tant que produits actifs sur la peau, à la fois en cosmétologie et en dermatologie, lesquels produits sont sélectionnés parmi :
- les didéhydrochlorophylles de formule Ib, dans lesquelles R₁ représente un groupe CH₃ (didéhydrochlorophylle a) ou un groupe CHO (didéhydrochlorophylle b), et R₄ représente un groupe phytyle,
- les didéhydrochlorophyllides de formule Ib, dans lesquelles R₁ a la même signification que pour les didéhydrochlorophylles et R₄ représente un atome d'hydrogène,
- les didéhydrophaéophytines de formule Ia, dans lesquelles R₁ et R₄ ont la même signification que pour les didéhydrochlorophylles, et
- les didéhydrophaéophorbides de formule Ia, dans lesquelles R₁ et R₄ ont la même signification que pour les didéhydrochlorophyllides.

2. Procédé d'obtention de l'ensemble des substances à noyau porphinique de formule Ia et Ib selon la revendication 1, caractérisé en ce qu'il comprend les étapes suivantes :
(1) au moins une macération à froid des parties de plantes chlorophylliennes et notamment de cyanophycées lyophilisées dans un solvant anhydre, choisi parmi le chloroforme, le chlorure de méthylène, l'éthanol absolu et le méthanol absolu, pour l'obtention d'un extrait contenant à la fois les didéhydrochlorophylles, les didéhydrochlorophyllides, les didéhydrophaéophorbides et les didéhydrophaéophytines, et
(2) séparation chromatographique de ces différentes substances en présence d'un solvant de polarité croissante.

3. Procédé selon la revendication 2, caractérisé en ce que le solvant à polarité croissante est choisi parmi l'acétone, le méthanol et l'éthanol plus ou moins aqueux.

4. Procédé selon la revendication 2 ou la revendication 3, caractérisé en ce qu'il comprend, préalablement à l'étape (1), un prétraitement de la plante chlorophyllienne, comprenant au moins une macération desdites plantes fraîches ou préalablement lyophilisées, dans un solvant très peu polaire, puis le séchage desdites plantes prétraitées.

5. Procédé selon la revendication 4, caractérisé en ce que le solvant peu polaire est choisi parmi l'éther de pétrole, le cyclohexane, l'hexane et le tétrachlorure de carbone.

6. Procédé de séparation spécifique des didéhydrochlorophyllides de formule Ib selon la revendication 1, caractérisé en ce qu'il comprend :
(1) au moins une macération de 24 h à une semaine des parties vertes de plantes chlorophylliennes et notamment de cyanophycées lyophilisées dans un solvant aqueux constitué de 20 à 60 % d'eau et d'un solvant miscible à l'eau choisi dans le groupe constitué par l'acétone, l'alcool éthylique et le méthanol,
(2) séchage des plantes ainsi traitées,
(3) au moins une macération à froid des plantes obtenues en (2) dans un solvant anhydre, pour l'obtention d'un extrait contenant essentiellement des didéhydrochlorophyllides et
(4) séparation chromatographique des didéhydrochlorophyllides.

7. Procédé de séparation spécifique des didéhydrophaéophorbides de formule Ia selon la revendication 1, caractérisé en ce qu'il comprend :
(1) une macération pendant 24 heures à une semaine à température ambiante d'un extrait de didéhydrochlorophyllides selon la revendication 6 dans un solvant organique auquel on a ajouté un acide miscible (acide formique, acide acétique ou autre acide à une concentration correspondant à un pH compris entre 2 et 4, en référence à une solution aqueuse) et
(2) séparation chromatographique des didéhydrophaéophorbides.

8. Procédé de séparation spécifique des didéhydrophaéophorbides de formule Ia selon la revendication 1, caractérisé en ce qu'il comprend :
(1) au moins une macération à température ambiante de 24 h à 48 h des parties vertes de plantes chlorophylliennes et notamment de cyanophycées lyophilisées dans un solvant miscible à l'eau tel que l'acétone, auquel on a ajouté un acide miscible (acide formique, acide acétique ou autre acide à une concentration correspondant à un pH compris entre 2 et 4, en référence à une solution aqueuse)
(2) séchage des plantes ainsi traitées,
(3) au moins une macération à froid des plantes obtenues en (2) dans un solvant anhydre, pour l'obtention d'un extrait contenant essentiellement des didéhydrophaéophorbides et
(4) séparation chromatographique des didéhydrophaéophorbides.

9. Procédé de séparation spécifique des didéhydrophaéophytines de formule Ia, caractérisé en ce qu'il comprend :
(1) une macération pendant une période de 24 h à une semaine, à température ambiante d'un extrait de didéhydrochlorophylles selon la revendication 2, dans un solvant organique auquel on a ajouté un acide miscible (acide formique, acide acétique..., à une concentration correspondant à un pH compris entre 2 et 4, en référence à une solution aqueuse) et
(2) séparation chromatographique des didéhydrophaéophytines.

10. Procédé selon l'une quelconque des revendications 6 à 9, caractérisé en ce qu'il comprend, préalablement à l'étape (1), un prétraitement de la plante chlorophyllienne, comprenant au moins une macération desdites plantes fraîches ou préalablement lyophilisées, dans un solvant très peu polaire, puis le séchage desdites plantes prétraitées.

11. Procédé selon l'une quelconque des revendications 2 à 10, caractérisé en ce que ladite cyanophycée est une souche de *Plectonema.*

12. Souche de *Plectonema,* caractérisée en ce qu'elle comprend l'ensemble des substances actives sur la peau selon la revendication 1.

13. Souche de *Plectonema* selon la revendication 12, déposée auprès de la Collection Nationale de Culture de Microorganismes de l'INSTITUT PASTEUR, 25 rue du Docteur Roux, 75724 PARIS Cédex 15 (FRANCE), en date du 29 mai 1991 sous le n° I-1101.

14. Utilisation d'une substance selon la revendication 1 pour l'obtention d'un médicament destiné à l'utilisation dermatologique.

15. Application à titre de produit cosmétique d'une substance selon la revendication 1.

16. Méthode de traitement esthétique de l'Homme dans la protection de la peau et la lutte contre le vieillissement naturel de la peau, comprenant l'administration d'au moins une substance selon la revendication 1, éventuellement associée à au moins un véhicule acceptable.

17. Méthode selon la revendication 16, caractérisée en ce que lesdites substances de formules Ia et Ib sont sous la forme d'une composition comprenant 0,1 à 3 % en poids desdites substances.

## Patentansprüche

1. Familie von Substanzen mit einem Porphyrinkern der folgenden allgemeinen Formeln Ia und Ib 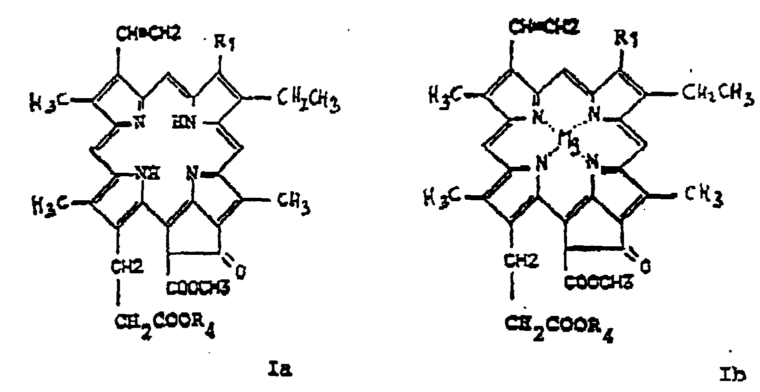 worin
. R₁ für eine Methylgruppe oder eine CHO-Gruppe steht, und
. R₄ für ein Wasserstoffatom oder eine Phytylgruppe steht
als auf der Haut sowohl kosmetisch als auch dermatologisch wirksame stoffe, ausgewählt aus der Gruppe
- Didehydrochlorophylle der Formel Ib, worin R₁ für eine CH₃-Gruppe (Didehydrochlorophyll a) oder eine CHO-Gruppe (Didehydrochlorophyll b) und R₄ für eine Phytylgruppe steht,
- Didehydrochlorophyllide der Formel Ib, worin R₁ die gleiche Bedeutung wie für die Didehydrochlorophylle hat und R₄ für ein Wasserstoffatom steht,
- Didehydrophäophytine der Formel Ia, worin R₁ und R₄ die gleiche Bedeutung wie für die Didehydrochlorophylle haben und
- Didehydrophäophorbide der Formel Ia, worin R₁ und R₄ die gleiche Bedeutung wie für die Didehydrochlorophyllide haben.

2. Verfahren zur Herstellung der Gesamtheit der Substanzen mit Porphyrinkern der Formel Ia und Ib nach Anspruch 1, dadurch **gekennzeichnet,** daß es die folgenden Stufen umfaßt:
(1) mindestens eine Kaltmazeration der lyophylisierten Teile der Chlorophyll-haltigen Pflanzen, insbesondere der Cyanophyceae, in einem wasserfreien Lösungsmittel, ausgewählt aus Chloroform, Methylenchlorid, absolutem Ethanol und absolutem Methanol, zum Erhalt eines Extrakts, der gleichzeitig die Didehydrochlorophylle, die Didehydrochlorophyllide, die Didehydrophäophorbide und die Didehydrophäophytine enthält, und
(2) chromatographische Trennung der verschiedenen Substanzen in Gegenwart eines Lösungsmittels mit steigender Polarität.

3. Verfahren nach Anspruch 2, dadurch **gekennzeichnet,** daß man das Lösungsmittel mit steigender Polarität aus mehr oder weniger wäßrigem Aceton, Methanol und Ethanol auswählt.

4. Verfahren nach Anspruch 2 oder 3, dadurch **gekennzeichnet,** daß es vor der Stufe (1) eine Vorbehandlung der Chlorophyll-haltigen Pflanze umfaßt, die mindestens eine Mazeration der frischen oder vorher lyophylisierten Pflanzen in einem sehr wenig polaren Lösungsmittel und anschließend die Trocknung der vorbehandelten Pflanzen umfaßt.

5. Verfahren nach Anspruch 4, dadurch **gekennzeichnet,** daß man das wenig polare Lösungsmittel aus Petrolether, Cyclohexan, Hexan und Tetrachlorkohlenstoff auswählt.

6. Verfahren zur spezifischen Trennung der Didehydrochlorophyllide der Formel Ib nach Anspruch 1, dadurch **gekennzeichnet,** daß es
(1) mindestens eine Mazeration von 24 Stunden bis zu einer Woche der grünen Teile der lyophylisierten Chlorophyll-haltigen Pflanzen und insbesondere der Cyanophyceae in einem wäßrigen Lösungsmittel, das aus 20 bis 60 % Wasser und einem Lösungsmittel, das mit Wasser mischbar ist, ausgewählt aus der Gruppe, bestehend aus Aceton, Ethylalkohol und Methanol, besteht,
(2) das Trocknen der so behandelten Pflanzen,
(3) mindestens eine Kaltmazeration der bei (2) erhaltenen Pflanzen in einem wasserfreien Lösungsmittel zum Erhalt eines Extrakts, der im wesentlichen Didehydrochlorophyllide enthält, und
(4) die chromatographische Trennung der Didehydrochlorophyllide
umfaßt.

7. Verfahren zur spezifischen Trennung der Didehydrophäophorbide der Formel Ia nach Anspruch 1, dadurch **gekennzeichnet,** daß es
(1) eine Mazeration während 24 Stunden bis zu einer Woche bei Raumtemperatur eines Didehydrochlorophylliden-Extrakts, wie er gemäß Anspruch 6 erhalten worden ist, in einem organischen Lösungsmittel, dem eine mischbare Säure (Ameisensäure, Essigsäure oder eine andere Säure mit einer Konzentration, die einem pH von 2 bis 4 in bezug auf eine wäßrige Lösung entspricht) zugesetzt worden ist, und
(2) die chromatographische Trennung der Didehydrophäophorbide
umfaßt.

8. Verfahren zur spezifischen Trennung der Didehydrophäophorbide der Formel Ia nach Anspruch 1, dadurch **gekennzeichnet,** daß es
(1) mindestens eine 24- bis 48-stündige Mazeration der grünen Teile der lyophylisierten Chlorophyll-haltigen Pflanzen und insbesondere der Cyanophyceae bei Raumtemperatur in einem mit Wasser mischbaren Lösungsmittel wie Aceton, dem eine mischbare Säure (Ameisensäure, Essigsäure oder eine andere Säure mit einer Konzentration, die einem pH von 2 bis 4 in bezug auf eine wäßrige Lösung entspricht) zugesetzt worden ist,
(2) die Trocknung der so behandelten Pflanzen,
(3) mindestens eine Kaltmazeration der bei (2) erhaltenen Pflanzen in einem wasserfreien Lösungsmittel zur Herstellung eines Extrakts, der im wesentlichen Didehydrophäophorbide enthält, und
(4) die chromatographische Trennung der Didehydrophäophorbide
umfaßt.

9. Verfahren zur spezifischen Trennung der Didehydrophäophytine der Formel Ia, dadurch **gekennzeichnet,** daß es
(1) eine Mazeration während eines Zeitraums von 24 Stunden bis zu einer Woche bei Raumtemperatur eines Didehydrochlorophyll-Extrakts nach Anspruch 2 in einem organischen Lösungsmittel, dem eine mischbare Säure (Ameisensäure, Essigsäure, ... mit einer Konzentration, die einem pH von 2 bis 4 in bezug auf eine wäßrige Lösung entspricht) zugesetzt worden ist, und
(2) die chromatographische Trennung der Didehydrophäophytine
umfaßt.

10. Verfahren nach einem der Ansprüche 6 bis 9, dadurch **gekennzeichnet,** daß es vor der Stufe (1) eine Vorbehandlung der Chlorophyll-haltigen Pflanze umfaßt, die mindestens eine Mazeration der frischen oder vorher lyophylisierten Pflanzen in einem sehr wenig polaren Lösungsmittel und anschließend die Trocknung der vorbehandelten Pflanzen umfaßt.

11. Verfahren nach einem der Ansprüche 2 bis 10, dadurch **gekennzeichnet,** daß die Cyanophyceae ein Plectonema-Stamm ist.

12. Plectonema-Stamm, dadurch **gekennzeichnet**, daß er die Gesamtheit der auf der Haut wirksamen Substanzen nach Anspruch 1 enthält.

13. Plectonema-Stamm nach Anspruch 12, hinterlegt am 29. Mai 1991 bei der Collection Nationale de Culture de Microorganismes de l'INSTITUT PASTEUR, 25 rue du Docteur Roux, 75724 PARIS Cédex 15 (Frankreich), unter der Nummer I-1101.

14. Verwendung einer Substanz nach Anspruch 1 zur Herstellung eines Arzneimittels zur dermatologischen Verwendung.

15. Verwendung einer Substanz nach Anspruch 1 als Kosmetikum.

16. Verfahren zur Schönheitsbehandlung des Menschen zum Schutz der Haut und zur Bekämpfung des natürlichen Alterns der Haut, umfassend die Verabreichung mindestens einer Substanz nach Anspruch 1, gegebenenfalls zusammen mit mindestens einem annehmbaren Träger.

17. Verfahren nach Anspruch 16, dadurch **gekennzeichnet,** daß die Substanzen der Formeln Ia und Ib in der Form einer Zusammensetzung, die 0,1 bis 3 Gew.-% der Substanzen enthält, vorliegen.

## Claims

1. Family of porphin ring substances of the general formulas Ia and Ib below: 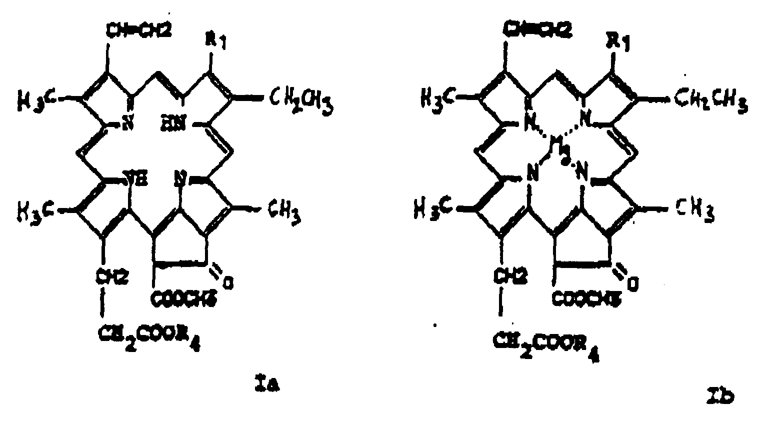 in which:
. R₁ is a methyl group or a CHO group; and
. R₄ represents a hydrogen atom or a phytyl group;
as products which are active on the skin, both cosmetically and dermatologically, which products are selected from among:
- didehydrochlorophyls of the formula Ib, in which R₁ represents a CH₃ group (didehydrochlorophyll a) or a CHO group (didehydrochlorophyll b), and R₄ represents a phytyl group;
- the didehydrochlorophylides of formula Ib, in which R₁ has the same meaning as for the didehydrochlorophyls and R₄ represents a hydrogen atom;
- the didehydrophaeophytines of formula Ia, in which R₁ and R₄ have the same meaning as in the case of the didehydrochlorophyles; and
- the didehydropheophorbides of formula Ia, in which R₁ and R₄ have the same meaning as for the didehydrochlorophylides.

2. Process of obtaining all these substances with a porphinic ring of formula Ia and Ib according to Claim 1, characterised in that it comprises the following steps:
(1) at least one cold maceration of parts of chlorophylous plants and in particular of freeze-dried cyanophytes in an anhydrous solvent, selected from among chloroform, methylene chloride, pure ethanol and pure methanol, to obtain an extract containing, at the same time, the didehydrochlorophyls, the didehydrochlorophylides, the didehydrophaeophorbides and the didehydrophaeophytines; and
(2) chromatographic separation of the different substances in the presence of a polar solvent.

3. Process according to Claim 2, characterised in that the polar solvent is selected from acetone, methanol and ethanol which are aqueous to a greater or lesser extent.

4. Process according to Claim 2 or Claim 3, characterised in that it comprises, before stage (1), a pretreatment of the chlorophylous plant, comprising at least one maceration of said fresh or previously freeze-dried plants in a solvent which is very slightly polar, and then the drying of said pretreated plants.

5. Process according to Claim 4, characterised in that the solvent which is very slightly polar is selected from among petroleum ether, cyclohexane, hexane and carbon tetrachloride.

6. Process of specific separation of didehydrochlorophylides of formula Ib according to Claim 1, characterised in that it comprises:
(1) at least one maceration of from 24 hours to one week of the green parts of chlorophylous plants and in particular of freeze-dried cyanophytes in an aqueous solvent consisting of from 20 to 60% of water and of a solvent which is miscible in water, selected from among the group consisting of acetone, ethyl alcohol and methanol;
(2) drying the plants which have been treated in this manner;
(3) at least one cold maceration of the plants obtained in stage (2) in an anhydrous solvent, to obtain an extract essentially containing didehydrochlorophylides; and
(4) chromatographic separation of didehydrochlorophylides.

7. Process of specific separation of the didehydrophaophorbides of formula Ia according to Claim 1, characterised in that it comprises:
(1) a maceration for from 24 hours to 1 week at an ambient temperature of an extract of didehydrochlorophylide, according to Claim 6, in an organic solvent to which a miscible acid (formic acid, acetic acid or another acid of a concentration corresponding to a pH of between 2 and 4, with reference to an aqueous solution) has been added; and
(2) chromatographic separation of the didehydrophaeophorbides.

8. Process of specific separation of the didehydrophaeophorbides of formula Ia according to Claim 1, characterised in that it comprises:
(1) at least one maceration at an ambient temperature of from 24 hours to 48 hours of the green parts of chlorophylous plants, and in particular of the freeze-dried cyanophytes, in a solvent which is miscible with water, such as acetone, to which there is added a miscible acid (formic acid, acetic acid or another acid of a concentration corresponding to a pH of between 2 and 4, with reference to an aqueous solution);
(2) drying the plants which have been treated in this manner;
(3) at least one cold maceration of the plants obtained in stage (2) in an anhydrous solvent, to obtain an extract essentially containing didehydrophaeophorbides; and
(4) chromatographic separation of the didehydrophaeophorbides.

9. Process of specific separation of the didehydropheophytins of formula Ia, characterised in that it comprises:
(1) a maceration for a period of from 24 hours to one week, at an ambient temperature, of an extract of didehydrochlorophylls according to Claim 2, in an organic solvent to which there has been added a miscible acid (formic acid, acetic acid, etc, at a concentration corresponding to a pH of between 2 and 4, with reference to an aqueous solution); and
(2) chromatographic separation of the didehydrophaeophytines.

10. Process according to any one of Claims 6 to 9, characterised in that it comprises, before stage (1), a pretreatment of the chlorophylous plant, comprising at least one maceration of said fresh or previously freeze-dried plants in a solvent with very low polarity, and then the drying of said pretreated plants.

11. Process according to any one of Claims 2 to 10, characterised in that the said cyanophyte is a strain of Plectonema.

12. Strain of Plectonema, characterised in that it comprises all the substances which are active on the skin according to Claim 1.

13. Strain of Plectonema according to Claim 12, deposited at the Collection Nationale de Culture de Microorganismes (National Collection of Microorganism Cultures) of INSTITUT PASTEUR, 25 rue du Docteur Roux, 75724 PARIS, Cedex 15 (FRANCE), on 29 May 1991 under No. 1-II01.

14. Use of a substance according to Claim 1 to obtain a medication for dermatological use.

15. Application of a substance according to Claim 1 as a cosmetic product.

16. Method of beauty treatment for human beings, for protecting skin and for combatting natural ageing of the skin, comprising the administration of at least one substance according to any one of Claims 1 to 6, optionally associated with at least one acceptable vehicle.

17. Method according to Claim 16, characterised in that said substances of formulas Ia and Ib are in the form of a composition comprising 0.1 to 3% by weight of said substances.
